# EUROPÄISCHE PATENTSCHRIFT

(11) **EP 0 464 648 B1**
(45) Veröffentlichungstag und Bekanntmachung des Hinweises auf die Patenterteilung: **06.12.1995**
(21) Anmeldenummer: 91110584.9
(22) Anmeldetag: 26.06.1991
(51) Int. Cl.: C07D 319/06, C07D 239/26, C07C 255/50, C09K 19/30, C09K 19/34

(54) **4-Cyano-3-fluorophenylester**
4-Cyano-3-fluorophenyl esters
Esters de 4-cyano-3-fluorophényl

(30) Priorität: 06.07.1990 CH 2254/90
(43) Veröffentlichungstag der Anmeldung: 08.01.1992
(73) Patentinhaber: F. HOFFMANN-LA ROCHE AG, 4002 Basel (CH)
(72) Erfinder: Buchecker, Richard, Dr., CH-8008 Zürich (CH); Schadt, Martin, Dr., CH-4411 Seltisberg (CH)
(74) Vertreter: Cottong, Norbert A.

(56) Entgegenhaltungen:
- DD-A- 252 828
- DE-A- 3 302 218
- DE-A- 3 515 633
- JP-B- 0 506 637

## Beschreibung

Die vorliegende Erfindung betrifft neue 4-Cyano-3-fluorophenylester, deren Herstellung, flüssigkristalline Gemische, die diese Verbindungen enthalten, sowie die Verwendung für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("super-birefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Flüssige Kristalle müssen eine gute chemische und thermische Stabilität und eine gute Stabilität gegenüber elektrischen Feldern und elektromagnetischer Strahlung besitzen. Flüssige Kristalle sollten zudem niedere Viskosität aufweisen und in den Zellen kurze Ansprechzeiten, tiefe Schwellenspannung und einen hohen Kontrast ergeben. Sie sollten eine geeignete Mesophase besitzen, beispielsweise für die obengenannten Zellen eine nematische oder cholesterische Mesophase. Weitere Eigenschaften, wie die elektrische Leitfähigkeit, wie dielektrische Anisotropie und die optische Anisotropie müssen je nach Zellentyp unterschiedlichen Anforderungen genügen. Es sind eine Vielzahl flüssigkristalliner Verbindungen bekannt. So sind bespielsweise in DE 3 515 633 und JP 66 377/1993 Benzonitrilderivate offenbart.

Flüssige Kristalle werden in der Regel als Mischungen eingesetzt, denn durch Mischung von verschiedenen Komponenten können die für die jeweilige Anwendung gewünschten Eigenschaften optimiert werden. Es ist daher wichtig, dass die Komponenten untereinander gut mischbar sind.

Gegenstand der Erfindung sind Verbindungen der allgemeinen Formel 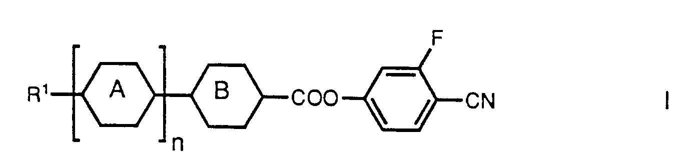
worin Ring A trans-1,4-Cyclohexylen, 1,4-Phenylen, Pyrimidin-2,5-diyl oder trans-1,3-Dioxan-2,5-diyl bezeichnet, wobei der Pyrimidin- bzw. der Dioxan-Ring jeweils in 2-Stellung mit dem Ring B verknüpft ist; n entweder 0 oder 1 ist; Ring B für 1,4-Phenylen, oder trans-1,4-Cyclohexylen steht; und R¹ 3E-Alkenyl oder an einem gesättigten Ring auch 1E-Alkenyl mit höchstens 12 Kohlenstoffatomen bedeutet, mit der Massgabe, dass R¹ 3-Butenyl ist, wenn n=0 ist und Ring B 1,4-Phenylen bedeutet.

Die Verbindungen der Formel I sind Flüssigkristalle, die einen überraschend breiten Mesophasen-Bereich und vergleichsweise hohe Klärpunkte aufweisen. Sie sind gut mit den üblichen Flüssigkristallmaterialien mischbar. Die genannten Verbindungen haben eine hohe positive dielektrische Anitotropie und dementsprechend niedrige Schwellenspannung. Die erfindungsgemässen Verbindungen eignen sich gut als Komponenten nematischer und cholesterischer Gemische und können dank ihrer guten Löslichkeit in vergleichsweise hohen Konzentrationen eingesetzt werden. Sie setzen in Gemischen dank ihrer hohen dielektrischen Anisotropie die Schwellenspannung herab, ohne die Mesophase einzuschränken.

Die Eigenschaften der Verbindungen der Formel I können je nach Zahl und Bedeutung der Ringe bis zu einem gewissen Grad variiert werden. Beispielsweise führen aromatische Ringe zu höheren und gesättigte Ringe zu tieferen Werten der optischen Anisotropie, Ringe wie Pyrimidin-2,5-diyl und trans-1,3-Dioxan-2,5-diyl beeinflussen die dielektrische Anisotropie, Verbindungen der Formel I, worin n=1 bedeutet, zeichnen sich zudem durch hohe Klärpunkte aus.

Der Ausdruck "Pyrimidin-2,5-diyl" bezeichnet einen Pyrimidinring, der in 2 und 5 Position substituiert ist. Der Ausdruck "trans-1,3-Dioxan-2,5-diyl" bezeichnet einen 1,3-Dioxanring der in 2 und 5 Position substituiert ist und dessen Substituenten trans zueinander stehen.

Der Ausdruck "gesättigter Ring" bezeichnet trans-1,4-Cyclohexylen und trans-1,3-Dioxan-2,5-diyl.

Der Ausdruck "3E-Alkenyl" umfasst geradkettige und verzweigte Gruppen wie beispielsweise 3-Butenyl, 3E-Pentenyl, 3E-Hexenyl, 3E-Heptenyl, 3E-Octenyl und dergleichen. Der Ausdruck "1E-Alkenyl" umfasst geradkettige und verzweigte Gruppen wie beispielsweise Vinyl, 1E-Propenyl, 1E-Butenyl, 1E-Pentenyl, 1E-Hexenyl, 1E-Heptenyl, 1E-Octenyl und dergleichen.

Die Formel I umfasst insbesondere Verbindungen der allgemeinen Formeln 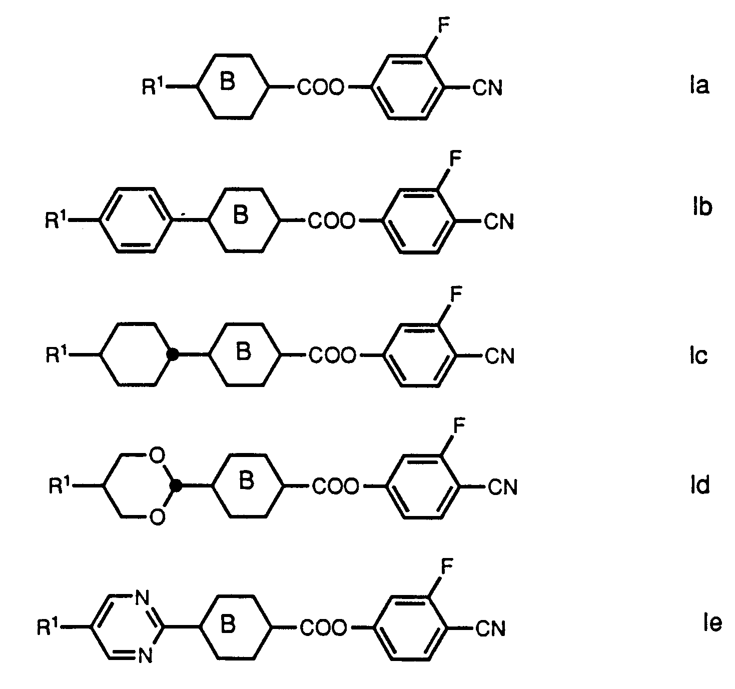
worin R¹ und Ring B obengenannte Bedeutungen haben.

Vorzugsweise stellt Ring B in den Formeln I und Ia-Ie 1,4-Phenylen dar.

R¹ besitzt in den obigen Formeln I und Ia-Ie jeweils höchstens 12 Kohlenstoffatome, besonders bevorzugte Reste R¹ sind diejenigen mit bis zu 7 Kohlenstoffatomen insbesondere diejenigen mit bis zu 5 Kohlenstoffatomen wie beispielsweise 3-Butenyl und 3E-Pentenyl und dergleichen, an einem gesättigten Ring auch Vinyl, 1E-Propenyl, 1E-Butenyl, 1E-Pentenyl und dergleichen.

Die Verbindungen der Formel I können erfindungsgemäss dadurch hergestellt werden, dass man eine Säure der allgemeinen Formel 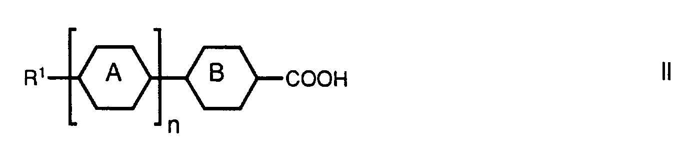
worin Ring A, Ring B, R¹ und n die oben genannte Bedeutung haben,
oder ein geeignetes Derivat dieser Säure mit 4-Cyano-3-fluorophenol verestert. Die Veresterung einer Säure der Formel II oder eines geeigneten Derivates beispielsweise Säurehalogenides, Säureesters, Anhydrides oder dergleichen kann auf an sich bekannte Weise erfolgen. Vorzugsweise kann eine Säure der allgemeinen Formel II in Gegenwart von N,N'-Dicyclohexylcarbodiimid und 4-(Dimethylamino)pyridin mit 4-Cyano-3-fluorophenol in bekannter Weise-beispielsweise wie in EP-A-0122389 beschrieben - umgesetzt werden.

Die Säuren der allgemeinen Formel II sind bekannt oder können beispielsweise aus bekannten Nitrilen durch Hydrolyse hergestellt werden (EP-A-0122389 und EP-A-0167912).

Die Erfindung betrifft ebenfalls flüssigkristalline Gemische mit mindestens zwei Komponenten, wovon mindestens eine Komponente eine Verbindung der Formel I ist.

Die Verbindungen der Formel I bewirken in den erfindungsgemässen flüssigkristallinen Gemischen, dass dank ihrer relativ hohen dielektrischen Anisotropie die Schwellenspannung sinkt, ohne dass der nematische Bereich eingeschränkt wird. Die erfindungsgemässen Verbindungen der Formel I eignen sich insbesondere für nematische Gemische mit hoher positiver dielektrischer Anisotropie und, sofern mindestens eine Komponente optisch aktiv ist, auch für cholesterische Gemische mit positiver dielektrischer Anisotropie. Sie eignen sich vor allem für die Anwendung in Zellen mit verdrillt nematischer Flüssigkristall-Struktur, insbesondere für STN-Zellen.

Aufgrund der guten Löslichkeit der Verbindungen der Formel I und aufgrund ihrer guten Mischbarkeit untereinander kann ihr Anteil in den erfindungsgemässen Gemischen relativ hoch sein. Im allgemeinen ist jedoch ein Anteil von 1-50 Gew.-%, insbesondere etwa 5-30 Gew.-% an Verbindungen der Formel I bevorzugt.

Ein bevorzugter Aspekt betrifft Gemische, welche neben einer oder mehreren Verbindungen der Formel I, eine oder mehrere Verbindungen der allgemeinen Formel 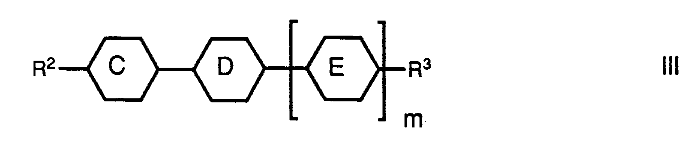
worin die Ringe C, D und E unabhängig voneinander 1,4-Phenylen, trans-1,4-Cyclohexylen oder einer der Ringe auch Pyrimidin-2,5-diyl bedeuten; m entweder 0 oder 1 ist; R² Alkyl, Alkoxyalkyl, 3E-Alkenyl, 4-Alkenyl oder an trans-1,4-Cyclohexylen auch 1E-Alkenyl bedeutet; und R³ Cyano, Alkyl, Alkoxy, Alkoxyalkyl, 3E-Alkenyl, 4-Alkenyl, 2E-Alkenyloxy, 3-Alkenyloxy oder an trans-1,4-Cyclohexylen auch 1E-Alkenyl bezeichnet,
und eine oder mehrere Verbindungen der allgemeinen Formel 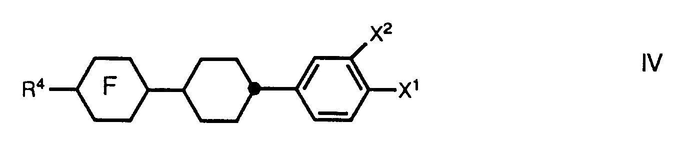
worin Ring F trans-1,4-Cyclohexylen oder trans-1,3-Dioxan-2,5-diyl bedeutet; R⁴ Alkyl, 1E-Alkenyl, 3E-Alkenyl, 4-Alkenyl oder Alkenyloxy bezeichnet; X¹ Fluor oder Chlor bedeutet; und X² Wasserstoff oder Fluor darstellt,
enthalten.

Vorzugsweise können diese flüssigkristalline Mischungen neben einer oder mehreren Verbindungen der Formel I einen Anteil an einer oder mehreren Verbindungen der allgemeinen Formel III, der vorzugsweise 20-80% beträgt enthalten. Besonders bevorzugt ist ein Anteil an einer oder mehreren Verbindungen der Formel III von 40-60%. Die flüssigkristallinen Mischungen können neben einer oder mehreren Verbindungen der Formel I sowie einer oder mehreren Verbindungen der allgemeinen Formel III einen Anteil von einer oder mehreren Verbindungen der allgemeinen Formel IV enthalten. Dieser Anteil beträgt vorzugsweise 5-25%, besonders bevorzugt beträgt der Anteil an einer oder mehreren Verbindungen der Formel IV 10-20%.

Weitere Verbindungen, die sich für Mischungen mit den erfindungsgemässen Verbindungen in hervorragender Weise eignen, sind Verbindungen der allgemeinen Formeln 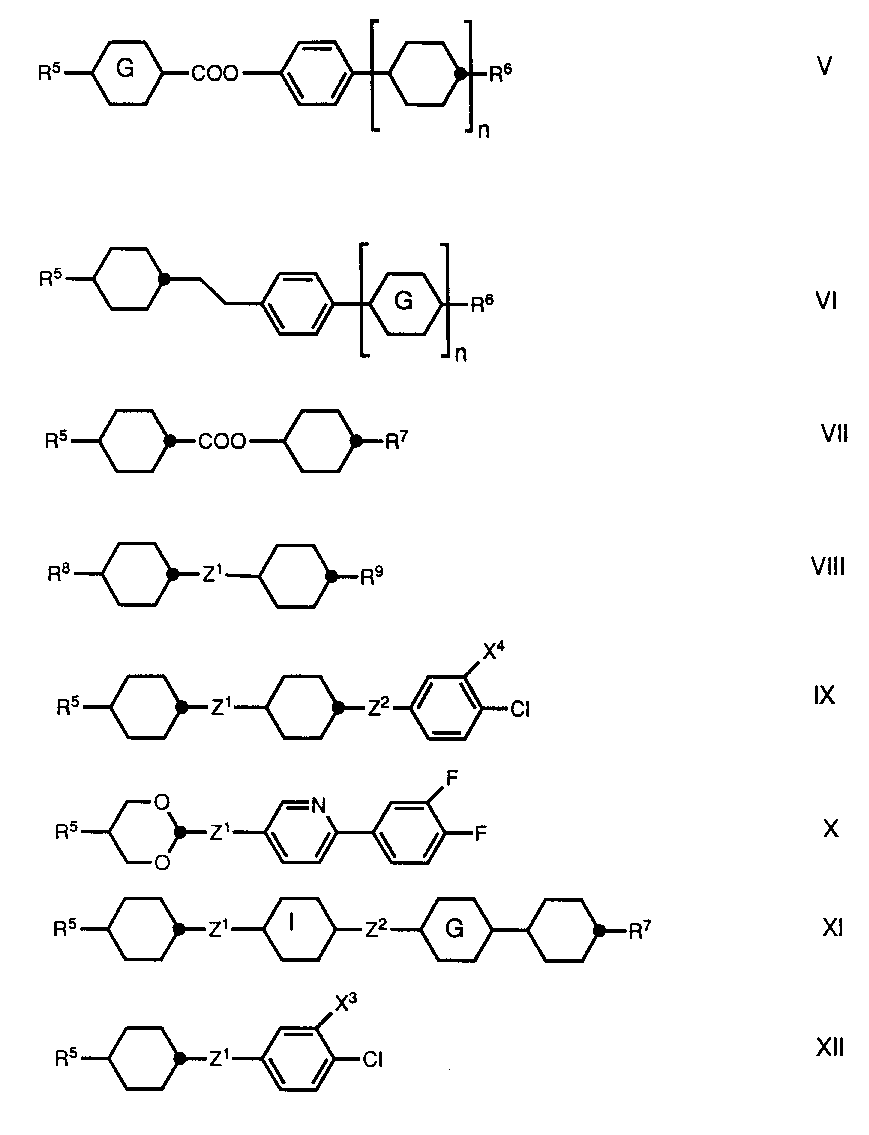 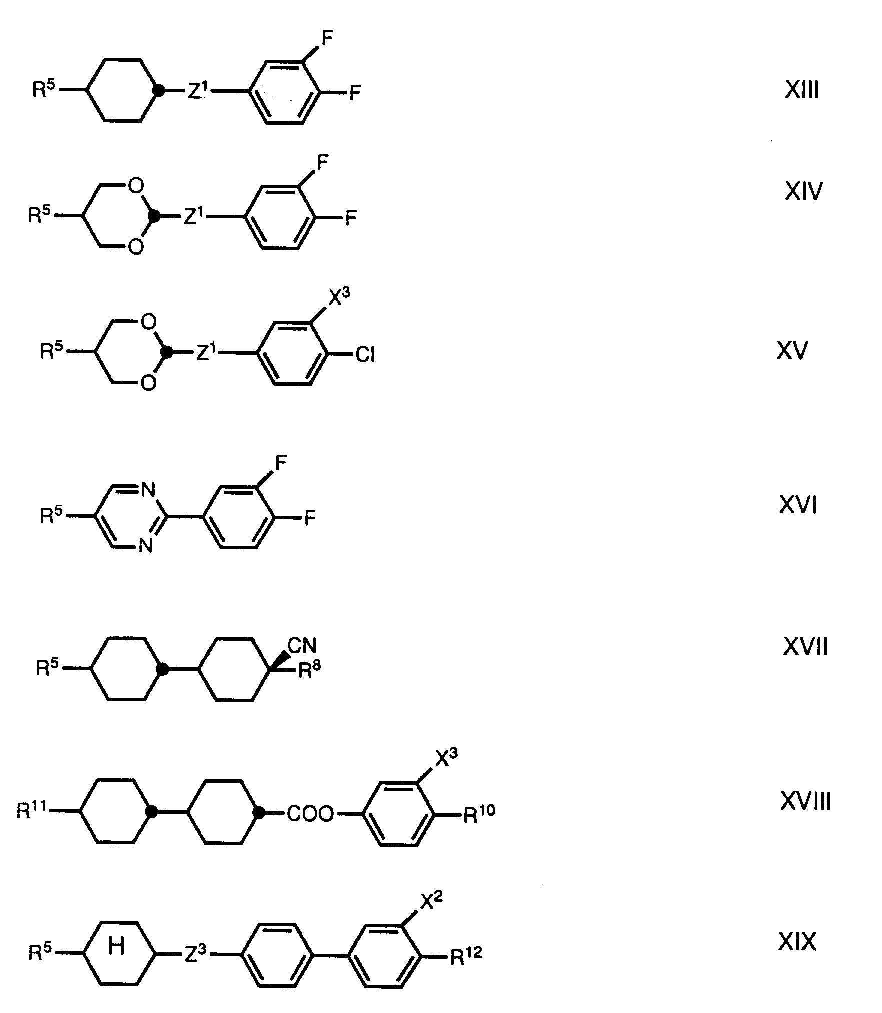
worin n für die Zahl 0 oder 1 steht; R⁵ und R⁷ unabhängig voneinander Alkyl, Alkoxyalkyl, 3E-Alkenyl, 4-Alkenyl oder an einem gesättigten Ring auch 1E-Alkenyl bedeutet; R⁶ Alkyl, 3E-Alkenyl, 4-Alkenyl oder an einem Cyclohexanring auch 1E-Alkenyl oder an einem Benzolring auch Cyano, Isothiocyanato, Alkoxy, 2E-Alkenyloxy oder 3-Alkenyloxy bedeutet; Ring G 1,4-Phenylen oder trans-1,4-Cyclohexylen darstellt; R⁸ Alkyl, 1E-Alkenyl, 3E-Alkenyl oder 4-Alkenyl bezeichnet; Z¹ und Z² jeweils eine einfache Bindung oder Aethylen darstellt, wobei zwei aromatische Ringe nur durch eine einfache Bindung gebunden sind; R⁹ Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxy-methyl bedeutet; Ring I 1,4-Phenylen, trans-1,4-Cyclohexylen, Pyridin-2,5-diyl, Pyrimidin-2,5-diyl oder trans-1,3-Dioxan-2,5-diyl darstellt; Ring H trans-1,4-Cyclohexylen oder trans-1,3-Dioxan-2,5-diyl; R¹⁰ Fluor, Chlor oder Cyano bedeutet; R¹¹ Alkyl, 1E-Alkenyl, 3E-Alkenyl oder 4-Alkenyl darstellt; R¹² Fluor oder Chlor darstellt; X² Wasserstoff oder Fluor bedeutet; X³ Wasserstoff, Fluor oder Chlor darstellt.

Der Ausdruck "gesättigter Ring" umfasst trans-1,4-Cyclohexylen und trans-1,3-Dioxan-2,5-diyl. Reste R⁵ bis R⁹ und R¹¹ besitzen vorzugsweise höchstens je 12 Kohlenstoffatome. Geradkettige Reste sind im allgemeinen bevorzugt.

Die Herstellung der Verbindungen der Formel I sowie flüssigkristalline Gemische enthaltend diese Verbindungen werden durch die folgenden Beispiele weiter veranschaulicht. C bedeutet eine kristalline, N eine nematische und I eine isotrope Phase. V₁₀ bezeichnet die Spannung für 10% Transmission, tₒₙ und t_{off} bezeichnen die Einschaltzeit bzw. die Ausschaltzeit und Δn bezeichnet die optische Anisotropie. Die elektro-optischen Eigenschaften wurden bei 22°C gemessen, sofern nichts anderes angegeben ist.

### Beispiel 1

Eine Suspension von 2,68 g 4-[trans-4-(1E-Propenyl)cyclohexyl]benzoesäure, 1,8 g 4-Cyano-3-fluorophenol und 187 mg 4-(Dimethylamino)pyridin in 25 ml Methylenchlorid wurde auf 0°C gekühlt und unter Rühren mit 3,17 g N,N'-Dicyclohexylcarbodiimid versetzt. Nach 5 Minuten Rühren bei 0°C und 3 Stunden bei Raumtemperatur wurde die Suspension filtriert und das Filtrat eingeengt. Chromatographische Reinigung des Rückstandes an 50 g basischem Aluminiumoxid mit Methylenchlorid und anschliessende Kristallisation aus Hexan/Aethylacetat ergab 2,67 g 4-Cyano-3-fluorophenyl-4-[trans -4-(1E-propenyl)cyclohexyl]benzoat als farblose Kristalle; Smp. (C-N) 117,5°C, Klp. (N-I) 233,1°C.

Auf analoge Weise können hergestellt werden:
4-Cyano-3-fluorophenyl-4-(3-butenyl)benzoat, Smp. (C-I) 68,6°C, Klp. (N-I) 37°C,
4-Cyano-3-fluorophenyl-4-[4'-(3-butenyl)biphenyl]carboxylat
4-Cyano-3-fluorophenyl-4-[4'-(3E-pentenyl)biphenyl]carboxylat
4-Cyano-3-fluorophenyl-4-[trans-4-(3-butenyl)cyclohexyl]benzoat
4-Cyano-3-fluorophenyl-4-[trans-4-(3E-pentenyl)cyclohexyl]benzoat
4-Cyano-3-fluorophenyl-4-(trans-4-vinylcyclohexyl)benzoat
4-Cyano-3-fluorophenyl-4-[trans-5-vinyl-(1,3-dioxan-2-yl)]benzoat
4-Cyano-3-fluorophenyl-4-[trans-5-(3-butenyl)-(1,3-dioxan-2-yl)]benzoat
4-Cyano-3-fluorophenyl-4-[trans-5-(3E-pentenyl)-2-(1,3-dioxan-2-yl)]benzoat
4-Cyano-3-fluorophenyl-4-[trans-5-(1E-propenyl)-2-(1,3-dioxan-2-yl)]benzoat, Smp. (C-N) 122,6°C, Klp. (N-I) 242,5°C.
4-Cyano-3-fluorophenyl-4-[5-(3-butenyl)-2-pyrimidyl]benzoat
4-Cyano-3-fluorophenyl-4-[5-(3E-pentenyl)-2-pyrimidyl]benzoat
4-Cyano-3-fluorophenyl-trans-4-vinylcyclohexancarboxylat
4-Cyano-3-fluorophenyl-trans-4-(1E-propenyl)cyclohexancarboxylat
4-Cyano-3-fluorophenyl-trans-4-(3-butenyl)cyclohexancarboxylat
4-Cyano-3-fluorophenyl-trans-4-(3E-pentenyl)cyclohexancarboxylat
4-Cyano-3-fluorophenyl-trans-4'-vinylbicyclohexylcarboxylat
4-Cyano-3-fluorophenyl-trans-4'-(1E-propenyl)bicyclohexylcarboxylat
4-Cyano-3-fluorophenyl-trans-4'-(3-butenyl)bicyclohexylcarboxylat
4-Cyano-3-fluorophenyl-trans-4'-(3E-pentenyl)bicyclohexylcarboxylat
4-Cyano-3-fluoropheny-trans-4-[4-(3-butenyl)phenyl]cyclohexancarboxylat
4-Cyano-3-fluorophenyl-trans-4-[4-(3E-pentenyl)phenyl]cyclohexancarboxylat
4-Cyano-3-fluorophenyl-trans-4-[trans-5-vinyl-2-(1,3-dioxan-2-yl)]cyclohexancarboxylat
4-Cyano-3-fluorophenyl-trans-4[trans-5-(1E-propenyl)-2-(1,3-dioxan-2-yl)]cyclohexancarboxylat
4-Cyano-3-fluorophenyl-trans-4-[trans-5-(3-butenyl)-2-(1,3-dioxan-2-yl)]cyclohexancarboxylat
4-Cyano-3-fluorophenyl-trans-4-[trans-5-(3E-pentenyl)-2-(1,3-dioxan-2-yl)]cyclohexancarboxylat
4-Cyano-3-fluorophenyl-trans-4-[5-(3-butenyl)-2-pyrimidinyl]cyclohexancarboxylat
4-Cyano-3-fluorophenyl-trans-4-[5-(3E-pentenyl)-2-pyrimidinyl]cyclohexancarboxylat.

Die als Ausgangsmaterial verwendete 4-[trans-4-(1E-Propenyl)cyclohexyl]-benzoesäure wurde wie folgt hergestellt:
Ein Gemisch von 2,5 g 4-[trans-4-(1E-Propenyl)cyclohexyl)]benzonitril und 6,22 g Kaliumhydroxid in 50 ml Diäthylenglykol wurde während 2,5 Stunden bei 180°C gerührt, abgekühlt, auf Eiswasser/Methylenchlorid gegeben und mit 25-proz. Salzsäure sauer gestellt. Nach Extraktion des Produktes mit Methylenchlorid, mehrmaligem Waschen der organischen Phase, Trocknen über Magnesiumsulfat wurde die organische Phase eingedampft und ergab 2,68 g 4-[trans-4-(1E-Propenyl)cyclohexyl]benzoesäure als farblosen Festkörper.

### Beispiel 2

Die Eigenschaften der Verbindungen wurden untersucht, indem binäre Gemische mit 4-(trans-4-Pentylcyclohexyl)benzonitril hergestellt wurden, deren Schwellenspannung und Ansprechzeiten in einer TN-Zelle (low bias tilt) mit 8 »m Plattenabstand gemessen wurden; als Betriebsspannung wurde der 2,5-fache Wert der Schwellenspannung V₁₀ gewählt. Die entsprechenden Daten für reines 4-(trans-4-Pentylcyclohexyl)benzonitril betragen: Klp. (N-I) 54,6°C, V₁₀=1,62V, tₒₙ=30 ms, t_{off}=A2 ms, Δn = 0,120.

### Mischung A

90 Gew.-% 4-(trans-4-Pentylcyclohexyl)benzonitril,
10 Gew.-% 4-Cyano-3-fluorophenyl-4-[trans-4-(1E-propenyl)-cyclohexyl]benzoat
Klp. (N-I) 64,2°C, V₁₀=1,46V, tₒₙ=31 ms, t_{off}=49 ms.

### Mischung B

80 Gew.-% 4-(trans-4-Pentylcyclohexyl)benzonitril,
20 Gew.-% 4-Cyano-3-fluorophenyl-4-[trans-4-(1E-propenyl)-cyclohexyl]benzoat
Klp. (N-I) 76,9°C, V₁₀=1,45V, tₒₙ=41 ms, t_{off}=60 ms.

### Mischung C

90 Gew.-% 4-(trans-4-Pentylcyclohexyl)benzonitril,
10 Gew.-% 4-Cyano-3-fluorophenyl-(3-butenyl)benzoat
Klp. (N-I) 50,8°C, V₁₀=1,24V, tₒₙ=35 ms, t_{off}=47 ms, Δn=0,127.

### Mischung D

80 Gew.-% 4-(trans-4-Pentylcyclohexyl)benzonitril,
20 Gew.-% 4-Cyano-3-fluorophenyl-(3-butenyl)benzoat
Klp. (N-I) 47,3°C, V₁₀=1,05V, tₒₙ=33 ms, t_{off}=55 ms, Δn=0,129.

### Beispiel 3

Die Verwendung der erfindungsgemässen Verbindungen wird in untenstehenden Mischungen exemplifiziert.

### Basis-Mischung 1

6,5% 4-(5-Butyl-2-pyrimidinyl)benzonitril.
7 % 4-[trans-4-(1E-Propenyl)cyclohexyl]benzonitril.
3 % 2-Cyano-5-(trans-4-butylcyclohexyl)pyrimidin.
3 % 2-Cyano-5-(trans-4-pentylcyclohexyl)pryrimidin.
2 % 2-(trans-4-Cyanocyclohexyl)-5-(trans-4-butylcyclohexyl)pyrimidin.
3 % 4-Cyano-4'-[trans-4-(1E-propenyl)cyclohexyl]biphenyl.
13 % trans-4-Vinyl-trans-4'-pentylbicyclohexyl.
13 % trans-4-(1E-Propenyl)-trans-4'-methoxymethylbicyclohexyl.
4 % trans-4-(3E-Pentenyl)-trans-4'-äthoxybicyclohexyl.
5 % trans-4-(3E-Pentenyl)-trans-4'-methoxybicyclohexyl.
5 % trans-(1E-Propenyl)-4'-(p-tolyl)bicyclohexyl.
8 % 4-[trans-4-(3E-Pentenyl)cyclohexyl]phenyl-(trans-4-propylcyclohexancarboxylat.
4 % 5-(trans-4-Pentylcyclohexyl)-2-[4-(trans-4-propylcyclohexyl)phenyl]pyrimidin.
4 % 4-Cyano-3-fluorophenyl-4-(3-butenyloxy)benzoat.
3 % trans-4-Vinyl-trans-4'-(4-fluorophenyl)bicyclohexyl.
3 % trans-4-(1E-Propenyl)-trans-4'-(4-fluorophenyl)bicyclohexyl.
3 % trans-4-(3-Butenyl)-trans-4'-(4-fluorophenyl)bicyclohexyl.
5 % 2-[trans-4-(4-Fluorophenyl)cyclohexyl]-5-(1E-propenyl)-1,3-dioxan.
5 % trans-4-(3,4-Difluorophenyl)-trans-4'-(1E-propenyl)bicyclohexyl.

### Basis-Mischung 2

2 % 4-(5-Butyl-2-pyrimidinyl)benzonitril.
8 % 4-[trans-4-(1E-Propenyl)cyclohexyl]benzonitril.
6 % 4-[trans-4-(3E-Pentenyl)cyclohexyl]benzonitril.
5 % 4-[trans-4-(1E-Butenyl)cyclohexyl]benzonitril.
5 % trans-4-Vinyl-trans-4'-cyanobicyclohexyl.
6,9 % trans-4-(1E-Propenyl)-trans-4'-cyanobicyclohexyl.
3 % 2-Cyano-5-(trans-4-butylcyclohexyl)pyrimidin.
3 % 2-Cyano-5-(trans-4-pentylcyclohexyl)pyrimidin.
6,4 % (p-Cyanophenyl)-4-[2-(trans-4-propylcyclohexyl)äthyl]-cyclohexylcarboxylat.
11 % trans-4-(3E-Pentenyl)-trans-4'-äthoxybicyclohexyl.
11,7% trans-4-Vinyl-trans-4'-pentylbicyclohexyl.
8 % 4-[trans-4-(3E-Pentenyl)cyclohexyl]phenyl-(trans-4-propylcyclohexancarboxylat.
3,9 % trans-4-Vinyl-4'-(4-fluorophenyl)bicylohexyl.
3,9 % trans-4-(1E-Propenyl)-4'-(4-fluorophenyl)bicylohexyl.
3,4 % p-Fluoro-phenyl-4-[trans-4-(3E-butenyl)cyclohexyl]cyclohexanoat.
6,8 % trans-4-(3,4-Difluorophenyl)-trans-4'-(1E-propenyl)bicyclohexyl.
6 % 2-[trans-4-(4-Fluorophenyl)cyclohexyl]-5-(1E-propenyl)dioxolan.

### Basis-Mischung 3

10 % 4-(5-Butyl-2-pyrimidinyl)benzonitril.
6 % 4-[trans-4-(1E-Propenyl)cyclohexyl]benzonitril.
5,5% 4-Cyano-4'-[trans-4-(1E-propenyl)cyclohexyl]biphenyl.
4,5% 4-Cyano-4'-[trans-4-(3E-pentenyl)cyclohexyl]biphenyl.
13 % trans-4-Vinyl-trans-4'-pentylbicyclohexyl.
13 % trans-4-(1E-Propenyl)-trans-4'-methoxymethylbicyclohexyl.
13 % trans-4-(3E-Pentenyl)-trans-4'-äthoxybicyclohexyl.
5 % trans-(1E-Propenyl)-trans-4'-(4-tolyl)bicyclohexyl.
4 % 4-[trans-4-(3E-Pentenyl)cyclohexyl)-4'-propylbiphenyl.
4 % 5-(trans-4-Pentylcyclohexyl)-2-[4-(trans-4-propylcyclohexyl)phenyl]pyrimidin.
7 % 4-Cyano-3-fluorophenyl-4-(3-butenyl)benzoat.
3,5% trans-4-Vinyl-trans-4'-(p-fluorophenyl)bicyclohexyl.
3,5% trans-4-(3-Butenyl)-trans-4'-(4-fluorophenyl)bicyclohexyl.
3,0% trans-4-(1E-Propenyl)-trans-4'-(4-fluorophenyl)bicyclohexyl.
5 % trans-4-(3,4-Difluorophenyl)-trans-4'-(1E-propenyl)bicyclohexyl.

### Mischungsbeispiel A

74,63 % Basis-Mischung 1
15,170% 4-Cyano-3-fluorophenyl-4-(3E-pentenyl)benzoat.
10,2 % 4-Cyano-3-fluorophenyl-4-(3-butenyl)benzoat.
Klp. (N-I) 84°C, V₁₀=1,3V, tₒₙ=30,3 ms, t_{off}=53 ms, Δn=0,131.

### Mischungsbeispiel B

74,91 % Basis-Mischung 2
15,1 % 4-Cyano-3-fluorophenyl-4-(3E-pentenyl)benzoat.
9,99 % 4-Cyano-3-fluorophenyl-4-(3-butenyl)benzoat.
Klp. (N-I) 82°C, V₁₀=1,32V, tₒₙ=35,5 ms, t_{off}=54,2 ms, Δn=0,125.

### Mischungsbeispiel C

80 % Basis-Mischung 3
12 % 4-Cyano-3-fluorophenyl-4-(3E-pentenyl)benzoat.
8 % 4-Cyano-3-fluorophenyl-4-(3-butenyl)benzoat.
Klp. (N-I) 93°C, V₁₀=1,47V, tₒₙ=22,4 ms, t_{off}=40,8 ms, Δn=0,144.

## Patentansprüche

1. Verbindungen der allgemeinen Formel 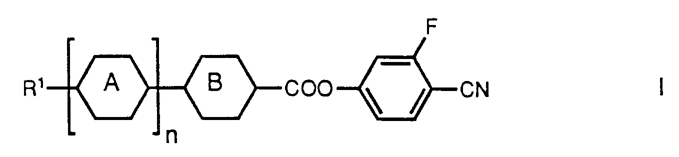 worin Ring A trans-1,4-Cyclohexylen, 1,4-Phenylen, Pyrimidin-2,5-diyl oder trans-1,3-Dioxan-2,5-diyl darstellt, wobei der Pyrimidin- bzw. der Dioxan-ring jeweils in Z-Stellung mit dem Ring B verknüpft ist; n entweder 0 oder 1 bedeutet; Ring B trans-1,4-Cyclohexylen oder 1,4-Phenylen darstellt; und R¹ 3E-Alkenyl oder an einem gesättigten Ring auch 1E-Alkenyl mit höchstens 12 Kohlenstoffatomen bedeutet, mit der Hassgabe, dass R¹ 3-Butenyl ist, wenn n=0 ist und Ring B 1,4-Phenylen bedeutet.

2. Verbindungen nach Anspruch 1, dadurch gekennzeichnet, dass R¹ höchstens 7 Kohlenstoffatome aufweist.

3. Verbindungen nach Anspruch 1 oder 2, gekennzeichnet durch die Formeln 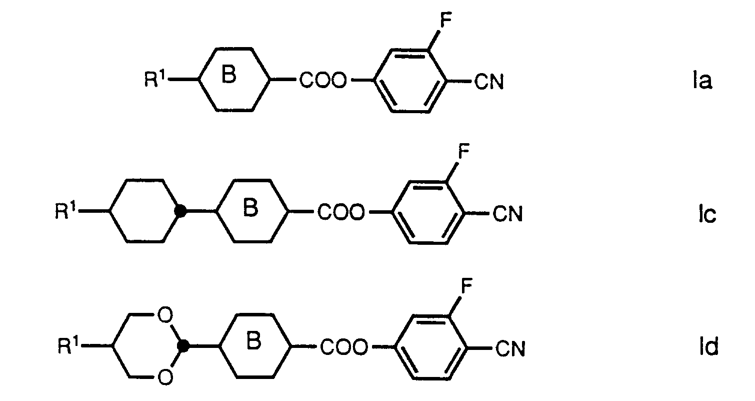 worin B 1,4-Phenylen bedeutet.

4. Flüssigkristallines Gemisch mit mindestens zwei Komponenten, dadurch gekennzeichnet, dass mindestens eine Komponente eine Verbindung der in Anspruch 1 definierten Formel I ist.

5. Flüssigkristallines Gemisch nach Anspruch 5, dadurch gekennzeichnet, dass der Anteil an Verbindungen der Formel I 1-50, vorzugsweise 5-30 Gew.-% beträgt.

6. Flüssigkristallines Gemisch nach Anspruch 4 oder 5, enthaltend mindestens eine Verbindung der allgemeinen Formel I, eine oder mehrere Verbindungen der allgemeinen Formel 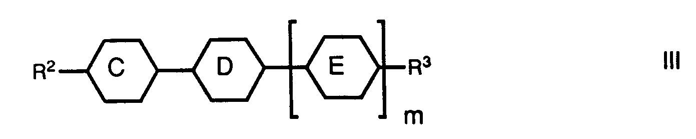 worin die Ringe C, D und E unabhängig voneinander 1,4-Phenylen, trans-1,4-Cyclohexylen oder einer der Ringe auch Pyrimidin-2,5-diyl bedeutet; m entweder 0 oder 1 ist; R² Alkyl, Alkoxyalkyl, 3E-Alkenyl, 4-Alkenyl oder an trans-1,4-Cyclohexylen auch 1E-Alkenyl bedeutet; und R³ Cyano, Alkyl, Alkoxy, Alkoxyalkyl, 3E-Alkenyl, 4-Alkenyl, 2E-Alkenyloxy, 3-Alkenyloxy oder an trans-1,4-Cyclohexylen auch 1E-Alkenyl bezeichnet,
und eine oder mehrere Verbindungen der allgemeinen Formel 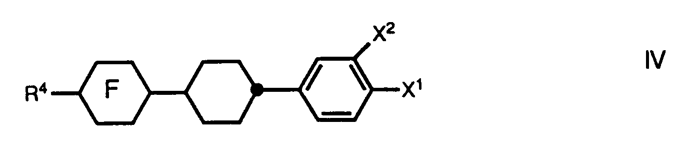 worin Ring F trans-1,4-Cyclohexylen oder trans-1,3-Dioxan-2,5-diyl bedeutet; R⁴ Alkyl, 1E-Alkenyl, 3E-Alkenyl, 4-Alkenyl oder Alkenyloxy bezeichnet; X¹ Fluor oder Chlor bedeutet; und X² Wasserstoff oder Fluor darstellt.

7. Flüssigkristallines Gemisch nach Anspruch 6 enthaltend einen Anteil von 20-80, vorzugsweise 40-60 Gew.-% an Verbindungen der Formel III und einen Anteil von 5-25, vorzugsweise 10-20 Gew.-% an Verbindungen der Formel IV.

8. Verfahren zur Herstellung der Verbindungen der in Anspruch 1 definierten Formel I, dadurch gekennzeichnet, dass man eine Säure der allgemeinen Formel 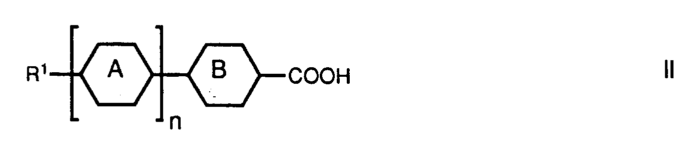 worin R¹, A, B und n die in Anspruch 1 angegebene Bedeutung haben,
oder ein geeignetes Derivat hiervon mit 4-Cyano-3-fluorophenol verestert.

9. Verwendung der Verbindungen der in Anspruch 1 definierten Formel I für elektro-optische Zwecke.

## Claims

1. Compounds of the general formula 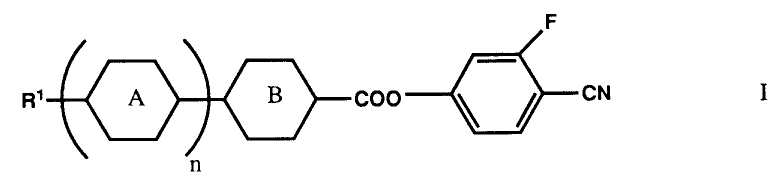 wherein ring A denotes trans-1,4-cyclohexylene, 1,4-phenylene, pyrimidine-2,5-diyl or trans-1,3-dioxane-2,5-diyl, with the pyrimidine ring and, respectively, the dioxane ring each being linked in the 2-position with ring B; n is either 0 or 1; ring B stands for 1,4-phenylene or trans-1,4-cyclohexylene; and R¹ signifies 3E-alkenyl or on a saturated ring also 1E-alkenyl having a maximum of 12 carbon atoms, with the proviso that R¹ is 3-butenyl when n is 0 and ring B signifies 1,4-phenylene.

2. Compounds according to claim 1, characterized in that R¹ has a maximum of 7 carbon atoms.

3. Compounds according to claim 1 or 2, characterized by the formulae 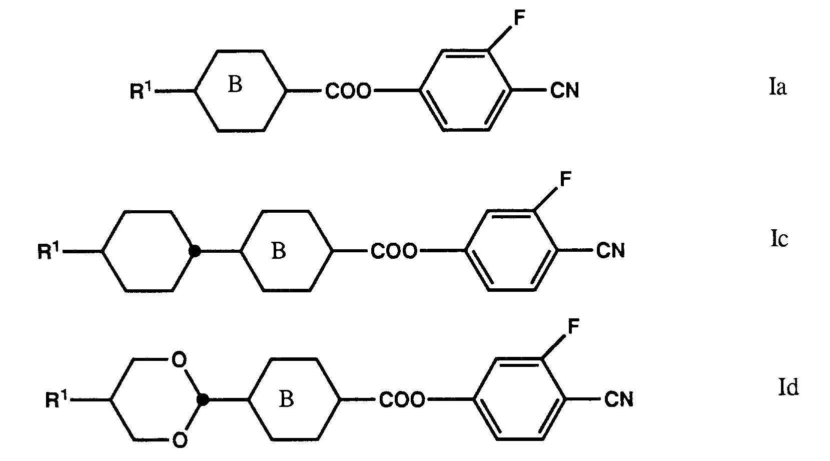 wherein B signifies 1,4-phenylene.

4. A liquid crystalline mixture having at least two components, characterized in that at least one component is a compound of formula I defined in claim 1.

5. A liquid crystalline mixture according to claim 5, characterized in that the amount of compounds of formula I is 1-50, preferably 5-30, wt.%.

6. A liquid crystalline mixture according to claim 4 or 5, containing at least one compound of general formula I, one or more compounds of the general formula 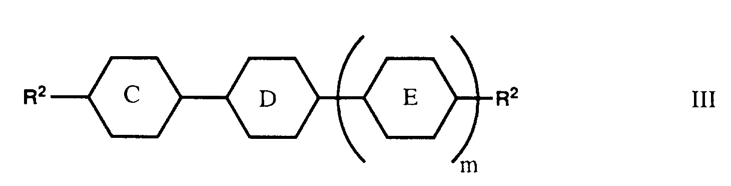 wherein rings C, D and E each independently signify 1,4-phenylene, trans-1,4-cyclohexylene or one of the rings also signifies pyrimidine-2,5-diyl; m is either 0 or 1; ring R² signifies alkyl, alkoxyalkyl, 3E-alkenyl, 4-alkenyl or on trans-1,4-cyclohexylene also 1E-alkenyl; and R³ denotes cyano, alkyl, alkoxy, alkoxyalkyl, 3E-alkenyl, 4-alkenyl, 2E-alkenyloxy, 3-alkenyloxy or on trans-1,4-cyclohexylene also 1E-alkenyl,
and one or more compounds of the general formula 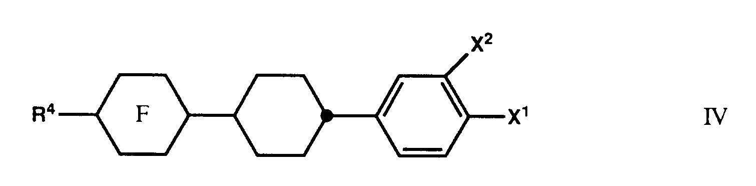 wherein ring F signifies trans-1,4-cyclohexylene or trans-1,3-dioxane-2,5-diyl; R⁴ signifies alkyl, 1E-alkenyl, 3E-alkenyl, 4-alkenyl or alkenyloxy; X¹ signifies fluorine or chlorine; and X² represents hydrogen or fluorine.

7. A liquid crystalline mixture according to claim 6 containing an amount of 20-80, preferably 40-60, wt. % of compounds of formula III and an amount of 5-25, preferably 10-20, wt.% of compounds of formula IV.

8. A process for the manufacture of the compounds of formula I defined in claim 1, characterized by esterifying an acid of the general formula 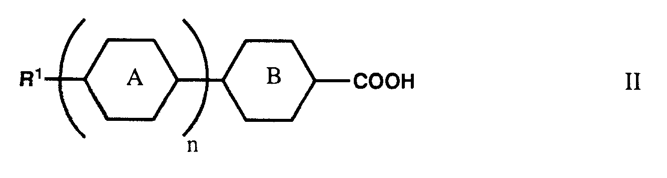 wherein R¹, A, B and n have the significance given in claim 1,
or a suitable derivative thereof with 4-cyano-3-fluorophenol.

9. The use of the compounds of formula I defined in claim 1 for electro-optical purposes.

## Revendications

1. Composés de formule générale 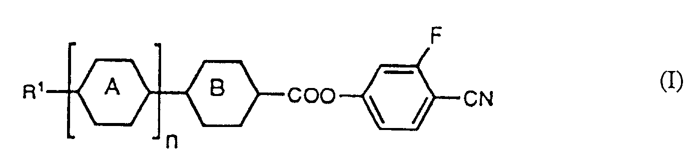 où le noyau A représente le trans-1,4-cyclohexylène, le 1,4-phénylène, le pyrimidine-2,5-diyle ou le trans-1,3-dioxane-2,5-diyle, le noyau pyrimidine ou dioxane étant relié en position 2 avec le noyau B ; n est égal à 0 ou 1 ; le noyau B représente le trans-1,4-cyclohexylène ou le 1,4-phénylène et R¹ représente un 3E-alcényle ou, sur un cycle saturé, également un 1E-alcényle ayant au plus 12 atomes de carbone, sous réserve que lorsque n est égal à 0 et que le noyau B est le 1,4-phénylène, R¹ est le 3-butényle.

2. Composés selon la revendication 1, caractérisés en ce que R¹ présente au plus 7 atomes de carbone.

3. Composés selon la revendication 1 ou 2, caractérisés par les formules 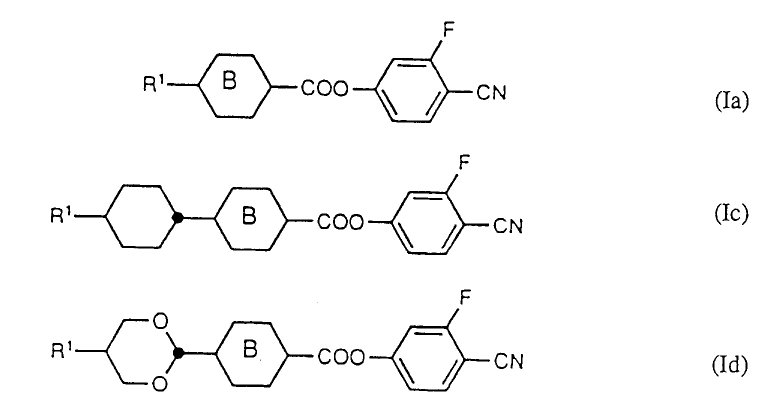 où B représente le 1,4-phénylène.

4. Mélange à cristaux liquides comportant au moins deux composants, caractérisé en ce qu'au moins un composant est un composé de formule I définie dans la revendication 1.

5. Mélange à cristaux liquides selon la revendication 4, caractérisé en ce que la proportion en composés de formule I est comprise entre 1 et 50 % en poids, de préférence entre 5 et 30 % en poids.

6. Mélange à cristaux liquides selon la revendication 4 ou 5, contenant au moins un composé de formule générale I, un ou plusieurs composés de formule générale 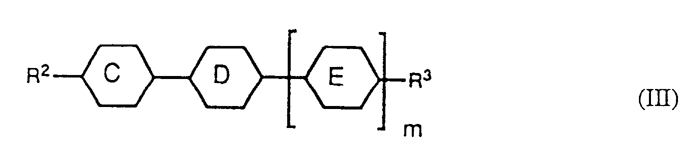 où les noyaux C, D et E représentent, indépendamment les uns des autres, le 1,4-phénylène, le trans-1,4-cyclohexylène ou l'un des noyaux représente également le pyrimidine-2,5-diyle ; m est égal à 0 ou 1; R² représente un alkyle, un alcoxyalkyle, un 3E-alcényle, un 4-alcényle ou, sur le trans-1,4-cyclohexylène, également le 1E-alcényle ; et R³ représente le cyano, un alkyle, un alcoxy, un alcoxyalkyle, un 3E-alcényle, un 4-alcényle, un 2E-alcényloxy, un 3-alcényloxy ou, sur le trans-1,4-cyclohexylène, également un 1E-alcényle, et un ou plusieurs composés de formule générale 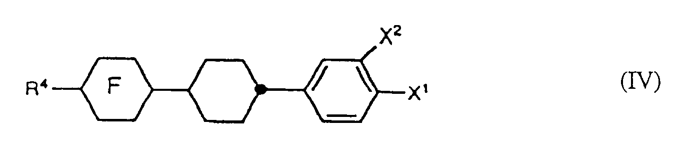 où le noyau F représente le trans-1,4-cyclohexylène ou le trans-1,3-dioxane-2,5-diyle ; R⁴ représente un alkyle, un 1E-alcényle, un 3E-alcényle, un 4-alcényle ou un alcényloxy ; X¹ représente le fluor ou le chlore; et X² représente l'hydrogène ou le fluor.

7. Mélange à cristaux liquides selon la revendication 6 contenant une proportion comprise entre 20 et 80 % en poids, de préférence entre 40 et 60 % en poids de composés de formule III et une proportion comprise entre 5 et 25 % en poids, de préférence entre 10 et 20 % en poids de composés de formule IV.

8. Procédé pour la préparation des composés de formule I définie dans la revendication 1, caractérisé en ce que l'on estérifie un acide de formule générale 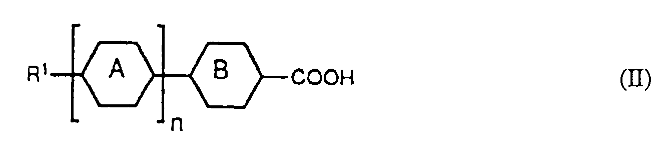 où R¹, A, B et n ont les significations données dans la revendication 1, ou l'un de ses dérivés appropriés avec le 4-cyano-3-fluorophénol.

9. Utilisation des composés de formule I définie dans la revendication 1 à des fins électro-optiques.
